(19) Europäisches Patentamt
European Patent Office
Office européen des brevets

(11) **EP 3 673 802 A1**

(12) **EUROPEAN PATENT APPLICATION**

(43) Date of publication:
**01.07.2020 Bulletin 2020/27**

(51) Int Cl.:
**A61B 5/08** *(2006.01)*  **A61B 5/107** *(2006.01)*
**A61B 5/113** *(2006.01)*  **A61B 5/00** *(2006.01)*

(21) Application number: **18248244.8**

(22) Date of filing: **28.12.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Nomics**
**4031 Liège (Angleur) (BE)**

(72) Inventors:
• **Ansay, Pierre**
 **4000 Liège (BE)**
• **Beckers, Bernard**
 **4920 Aywaille (BE)**

(74) Representative: **Calysta NV**
 **Lambroekstraat 5a**
 **1831 Diegem (BE)**

(54) **DISTANCE MEASURING DEVICE FOR DETECTING SLEEP DISORDERS**

(57) Sleep disorder detector comprising an emitter and a receiver, in which said emitter comprises an optimized energizing circuit, allowing very low power implementations and usage of a selected group of cables.

FIGURE 6

EP 3 673 802 A1

## Description

## Technical Domain

The present invention relates to a distance measurement device which is capable of very accurately measuring distances, in particular when mounted on a living being through a support, without having to use voltages or magnetic field values which would harm the human body.

## Technological Background of the invention

The invention concerns a distance measuring device comprising an emitter and a receiver, said emitter containing a resonance circuit having a resonance frequency in order to produce a magnetic field , said emitter being further arranged to produce a magnetic field emission intermittently, said emission having a predetermined energy, said receiver being connected to a conditioning and measuring unit comprising a detector, said receiver being further arranged to pick up at said resonance frequency the magnetic field emitted by said emitter, said receiver being further arranged to provide a first signal representing the voltage level associated to said picked up magnetic field emission to the input of said detector, said detector being arranged to determine a residual energy value by correlation of said first signal with a second predetermined signal having a waveform representative of a signal to be picked up by the receiver, said detector being further arranged to produce a distance measurement signal representing the distance between emitter and receiver, based on said residual energy value.

The invention also concerns a sleep disorder detector comprising a distance measuring device.

The sleep disorder detector is then mounted on a support arranged to be applied onto the head of a living being so as to measure movements of the mouth.

Signal processing techniques are then used so as to detect all sleep respiratory disorders. The starting point is the data from the detector and additional signals are derived by filtering. It is the combination of these signals that reveals the various sleep respiratory disorders: central apnea, mixed apnea, obstructive apnea, hypopnea, and upper airways resistance syndrome (UARS). Reference signal are attributed to events, such as mandibular movement of salient (MMS), lowering (MML) and opening (MMO) types.

## Prior art

Distance measurement Device as mentioned in the beginning are well known in the art, see for example in document to US 2007/0273366 A1 & DE 4114 398

Unfortunately, those devices present the drawback to use a relatively high voltage level to charge the resonance circuit. Said higher voltage results on one hand in a higher power consumption of the device, certainly when taking in account that this distance measurement is used to monitor the movement of the mouth of a living being, and so performed almost continuously with small time intervals in between.

## Brief summary of the invention

In a first aspect, the present invention relates to a distance measurement device.

In a second aspect, the present invention relates to a sleep disorder detector using said distance measurement device

In a third aspect, the present invention relates to a movement analyze comprising a distance measurement device according to the invention.

In a fourth aspect, the object of the invention relates to a method for measuring a distance for identifying a sleep disorder using the device according to the invention.

The invention concerns a distance measuring device comprising an emitter and a receiver, said emitter containing a resonance circuit having a resonance frequency, said emitter being further being arranged to produce a magnetic field emission intermittently, said emission having a predetermined energy, said receiver being connected to a conditioning and measuring unit comprising a detector, said receiver further arranged to pick up at said resonance frequency the magnetic field emitted by said emitter, said receiver being further arranged to provide a first signal representing the voltage level associated to said picked up magnetic field emission to the input of said detector, said detector being arranged to determine a residual energy value by correlation of said first signal with a second predetermined signal having a waveform representative of a signal to be picked up by the receiver, said detector being further arranged to produce a distance measurement signal representing the distance between emitter and receiver, based on said residual energy value.

The invention also concerns a sleep disorder detector comprising a distance measuring device.

Such a device is known from the patent application US 2007/0273366 A1. According to the known device, the strength of the measured magnetic field determines the distance between the emitter and the receiver and can be used to accurately measure the distance between 2 points.

One drawback of the known device is the high amount of energy required to charge the emitter circuit. As the invention is mainly used while mounted on the face of a living being, high energy values should be avoided due to safety risks. Secondly, this high energy values will also imply the use of strong shielded wiring between said emitter, said receiver and said detector in order to avoid interference with the measured signal.

The aim of the invention is to produce a distance measuring device that is capable of measuring distances very accurately, using a low magnetic field value, produced by a low voltage resonance circuit, without having a detrimental effect on the health of the human body.

To this end, a device according to the invention is characterized in that said resonance circuit of the emitter comprises an inductance and a capacitance, said resonance circuit being further connected to said conditioning and measuring unit comprising an energizing circuit, said energizing circuit comprising a first, a second, a third and a fourth transistor switch in series, said resonance circuit being connected to the energizing circuit, said capacitance of the resonance circuit being connected to the first and fourth switch of the energizing circuit on one side of the capacitance and to the inductance on the opposite side of the capacitance, said inductance of the resonance circuit being further connected to the second and third transistor switch, said energizing circuit being further connected to a voltage source between the fourth and third switches and connected to ground between the first and second transistor switches, said two circuits being arranged to switch between different charging states to create and amplify a magnetic field, generated by said resonance circuit, until the energy value of said magnetic field reaches the predefined maximum energy value, said emitter and said conditioning and measuring unit, said receiver and said conditioning and measuring unit being connected to each other by using a cable selected in the group consisting of shielded single core, coaxial, shielded twisted pair, twisted pair, ...

It has been indeed realized according to the present invention that it is possible through an optimized, more efficient charging circuit to solve at least a part of the previously stated drawbacks by providing a device in which the voltage used to charge the capacitor can be many times lower than the desired voltage level to reached across said capacitor.

As it can be seen, the distance measuring device according to the present invention the emitter comprises a resonance circuit and the conditioning and measuring unit comprises an energizing circuit. The specific arrangement of said resonance circuit and said energizing circuit allows said energizing circuit and said resonance circuit to switch between different charging states to create and amplify a magnetic field, generated by said resonance circuit. Therefore, even if the voltage source generates a low level voltage, this creates a magnetic field value which will be further amplified until the energy value of said magnetic field reaches the predefined maximum energy value. The amplification of the magnetic field by switching the 4 switches alternatively a predefined number of times in order to alternate between the different possible configuration of the resonance circuit makes it possible to use a low voltage, although providing enough resolution to the distance measuring device according to the present invention, in order to measure distances very accurately.

The distance measuring device is indeed intended to measure the movement of the mouth of a human being while sleeping. While some movements are with a quite big amplitude and are easy to detect, others are less easy to measure and require a high resolution of the distance measurement device. As mentioned in the prior art, to measure the breath effort upon sleeping, the distance measuring device should be able to measure very light mouth movement (less than 0,1 mm for mouth openings up to 5 cm compared with the mouth closed position) and this, regularly.

It has been now identified surprisingly, while the prior art was teaching to have a high voltage circuit to be able to reach enough accuracy for the measure, that it is also possible to reach a very high accuracy of the measure by using a low voltage input, which creates a the same magnetic field in the connections between emitter and respectively receptor and the detector if carefully selecting the right cable, to connect the elements without having detrimental effect of cross influence.

Indeed, as mentioned in US 2007/0273366, for matters of comfort, the cables are generally gathered together over the major part of their length, to separate only at the approach to the measuring elements.

Advantageously, in the distance measuring device according to the present invention, said detector is arranged to implement said correlation with said second signal, which second signal is formed by said waveform representing the signal to be picked up as obtained in the absence of perturbation. In a further preferred embodiment, said second signal can alternatively be formed by said waveform being represented by a sinusoidal waveform in synchronization with the first signal itself multiplied by a time window with reduced taper factor or by a square waveform in synchronization with the first signal.

Preferably, the emitter is housed in a protective case and arranged to produce said magnetic field outside said case with a strength less than 2 mTesla, preferably less than 1mTesla, such as less than 800 $\mu$Tesla, less than 600 $\mu$Tesla, less than 500 $\mu$Tesla, less than 100 $\mu$Tesla, more preferably less than 1 $\mu$Tesla.

Advantageously, said emitter further comprising an inductance and a capacitor connected in series with one another and connected by means of electrical conductors to said conditioning and measuring unit comprising said energizing circuit, said energizing circuit comprising a voltage source.

In particular, said energizing circuit being placed close to the inductance and the capacitor and forming an autonomous unit with respect to the receiver.

Other embodiments of the distance measuring device according to the present invention are mentioned in the appended claims.

The present invention also relates to a sleep disorder detector comprising a distance measuring device according to the present invention.

Preferably, the sleep disorder detector according to the present invention comprises a casing for being placed directly on the head of a living being as to measure the movement of the mouth.

Advantageously, said detector comprises an analyzer having an input connected to the distance measuring device and is arranged to receive (directly or later) said distance measurement, said analyzer can also be an external analyzer (i.e. a computer provided with the required software), said analyzer being arranged to compare and match the measured signal with a collection of characteristic value sets describing movement patterns of the mouth of a living being, and to identify a characteristic set of movement pattern within said collection characteristic value sets describing movement patterns, producing a detection signal upon.

Favorably, said characteristic value sets further comprises a first characteristic set of characteristic values describing a sudden closing of the mouth, a second characteristic set of characteristic values indicating a slow opening of the mouth, a third characteristic set of characteristic values indicating a slow closing of the mouth, and a fourth characteristic set of characteristic values indicating an increase in the amplitude of the signal at the breathing frequency followed by a decrease in the signal at the breathing frequency.

Preferably, said analyzer is arranged to add the distance measure signal to the characteristic set which matched to distance measure signal, to be used and to improve the matching to this pattern in the future. Said analyzer is further arranged to produce a specific sleep disorder signal upon recognition of a specific sequence of any of the first, second, third and fourth characteristic set of characteristic values.

Advantageously, said detector comprising a decision element using said detection signal to provide an indication of insufficient, correct or excessive treatment for the targeted sleep disorders.

Other embodiments of the sleep disorder detector according to the present invention are mentioned in the appended claims.

The present invention also relates to a movement analyzer comprising a distance measuring device according to the present invention, wherein said device is mounted on a support arranged to be applied around a joint of a living being as so to measure the characteristics and/or statistics of the movements of this joint, which can be to monitor the development of Parkinson's disease for example.

Other embodiments of the movement analyzer according to the present invention are mentioned in the appended claims.

The present invention further relates to a method for measuring a distance for identifying a sleep disorder comprising the steps of :

    i) Production of a magnetic field intermittently having a maximum predefined energy value by an emitter having a resonance circuit having a resonance frequency,
    ii) picking up by said receiver at said resonance frequency the magnetic field produced by the emitter, and providing the associated voltage level to the input of said conditioning and measuring unit comprising said detector, to whom said receiver is connected,
    iii) Determining by said detector based on the ratio between the maximum energy value and the residual energy value, a distance measurement signal representing the distance between said emitter and said receiver, said determining step of said distance measurement signal being done by correlation of said first signal with a second predetermined signal having a waveform representative of a signal to be picked up by the receiver, said second signal comprising a time window having a predetermined duration and comprising at least an initial sub-period and a final sub-period, said second signal being an alternating signal synchronized with the first signal and whereof the amplitude is attenuated during the initial and the final periods,

characterized in that said method further comprises a step of alternating opposite charging states of the resonance circuit, thereby creating and amplify a magnetic field, generated by said resonance circuit, until the energy value of said magnetic field reaches the predefined maximum energy value, said emitter and receiver being connected to said conditioning and measuring unit comprising said detector by using a cable selected in the group consisting of shielded single core, coaxial, shielded twisted pair, twisted pair, ...

The method further comprising a multiplication and integration with said second signal formed by said waveform representing the signal to be picked up as obtained in the absence of perturbation by said detector.

Other embodiments of the method according to the present invention are mentioned in the appended claims.

The invention will now be described in more detail with the help of the drawings that depict a preferential embodiment of a device according to the invention and a sleep disorder detector comprising a distance measuring device according to the invention. In the drawings:

    FIG 1: Illustrates the basic structure of the measuring device.
    FIG 2: Illustrates the energizing of the emitter and the signal emitted by the emitter and the signal received by the receiver.
    FIG 3: Illustrates the energizing topology for the resonant circuit, forming the emitter.
    FIG 4: Shows the different circuit states and the corresponding states of the switches.
    FIG 5: Shows a schematic representation of the energizing circuit of the emitter.
    FIG 6: Illustrates how the invention can be mounted on the face of a human being.

## DETAILED DESCRIPTION OF THE INVENTION

The distance measuring device uses a robust distance sensor for physiological use. Using a magnetic field to measure the distance offers many advantages, as it passes through clothing, pillows, sheets, ... The magnetic field being used for the measurement is extremely small, and periodic, at the measuring rate. The maximum amplitude reaches a value less than 1mTesla, considered to be non-harmful to health in the event of continuous exposure.

The sensor is designed to operate with loose magnetic coupling, which means that the emitter affects the receiver, but the attenuation between the emitter and the receiver does not allow the receiver to affect the emitter in return. The detection method is implemented in digital processing which allows very accurate measuring while still making use of small components, assuring an easy to implement ergonomic system.

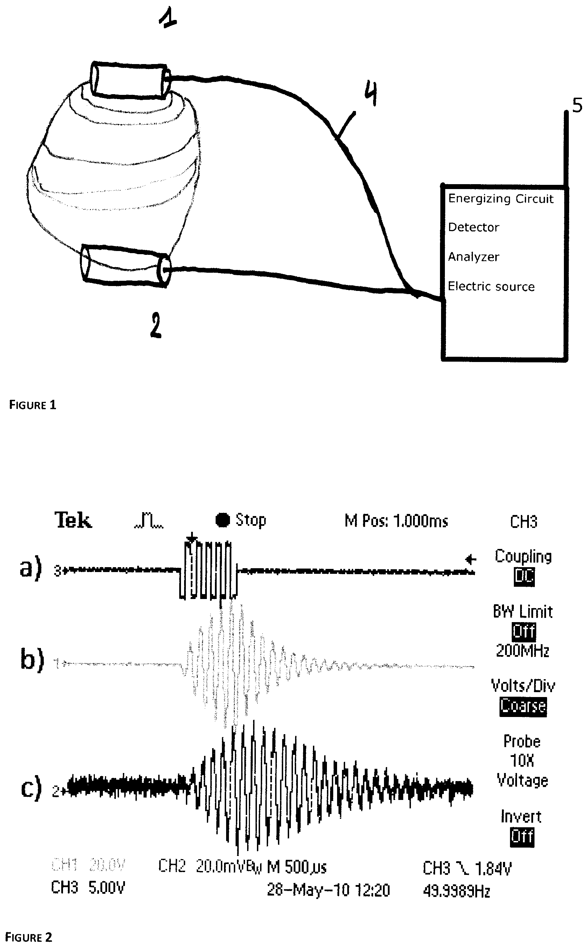

FIG. 1 illustrates an example of a basic structure of a device according to the invention. The device comprises an emitter 1 and a receiver 2 with a magnetic field being generated by said emitter, picked up by said receiver. A conditioning and measuring unit 5 comprising the detector, analyzer and an energizer circuit. The emitter 1 and the receiver using the principle of 2 resonance circuits that are able to influence each other if they have a mutual inductance. The use of these circuits significantly increases the accuracy of the distance measuring device. Due to the low voltage values required to charge the emitter circuit, the influence of said voltage on the distance measure signal produced by the receiver will be negligible.

In order to further reduce power consumption, the resonance circuit at said emitter side is only being loaded each time a measurement event takes place. A predefined series of voltage pulses will be created by the energizing circuit, alternating between positive and negative, in order to energize the emitter as shown in FIG. 2A. The current flowing in the emitting inductance will result in a magnetic field characterized in FIG 2B. The voltage obtained at the side of the receiver 2, in the event of compatibility of the resonant circuits, is shown on FIG 2C.

The envelope of this signal depends on the quality factor of the resonant circuits being used. In the event of poor compatibility of the resonant circuits, this envelope can be significantly modified, with the possible appearance of a beat phenomenon. The maximum value of the voltage observed at the receiver varies as a function of the distance between emission inductance and receiving inductance according to the relationship:

$$V = \left(\frac{\alpha}{d^3}\right) + \beta$$

Where a is the overall detection gain, including the effect of the amplifiers, β a possible offset due to the detection circuits and d the distance between the emitter and the receiver

In the preferred embodiment of the invention, the resonant circuits are tuned to a frequency of 5 to 8 kHz, so as to maximize the quality factor of the resonant circuits whilst remaining below the radio frequencies. The use of miniature inductances can however lead to the adoption of frequencies going up to 50, perhaps even 100 kHz, according to the capabilities of the detection circuit. The value and the size of the resonance inductances are adapted so as to be able to base the detection on a signal having 10 to 30 periods of resonance of significant amplitude. Shorter durations degrade the detection performance, whilst larger values require too great an accuracy, in practice, in the tuning of the resonant circuits.

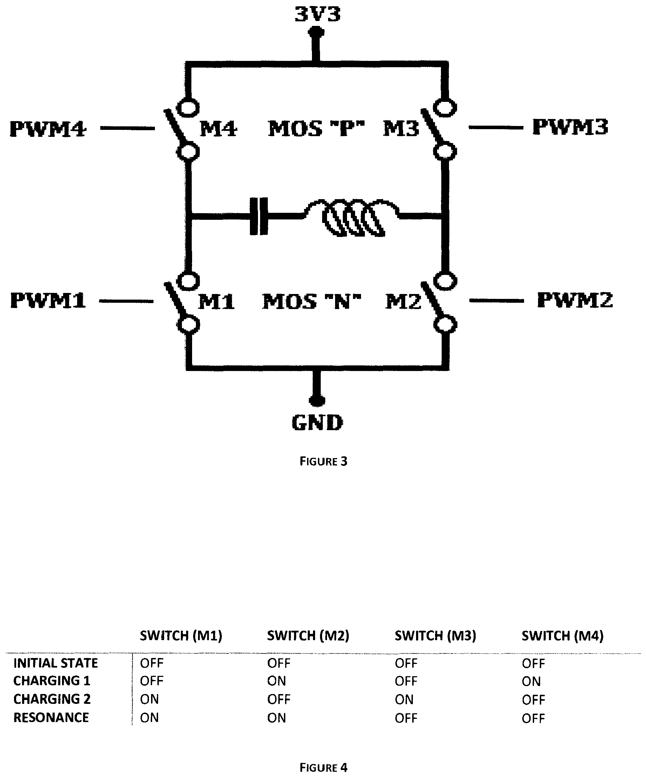

The schematic representation of the energizing circuit together with the resonant circuit is shown in FIG. 3. The 4 transistor switches (M1, M2, M3, M4) determine the state of the energizing circuit. The invention makes use of 4 possible states:

i) Initial state: all switches are in an off state, the circuit is neither energizing nor resonating.

ii) Charging state 1: Switches M2 & M4 are closed while M1 & M3 are open. The voltage source will allow the inductance to let current increase and hence capacitor to charge towards the supply voltage, causing the resonance circuit to start oscillating and creating a magnetic field as shown in FIG 2.

iii) Charging state 2: Switches M2 & M4 open, whilst M1 & M3 are closed. This will cause an inverted voltage to be applied to the dipole formed by capacitor and inductance, reversing the phenomenon of charging state 1, boosting the inversion of current in the inductance and the discharge of the capacitor.

iv) Resonance state: Switches M3 & M4 opened, whilst M1 & M2 are closed, the resonance circuit is completely coupled off the energizing circuit and will continue to oscillate until all energy is converted into a magnetic field.

The combination of energizing and resonance circuit will alter a predefined amount of times between charging state 1 & charging state 2, causing the amplitude of the magnetic field created by the inductance to reach the predefined value. This alternation between both charging states allows the circuit to reach a voltage level across the capacitor many times higher than the voltage level of the source. The energizing circuit comprised in the conditioning and measuring unit will alternate a predefined amount of times between charging state 1 and charging state 2, until the desired energy level has been reached (such as 15, 20, 25, ... times higher than the voltage level of the source). This lower charging voltage lowers the effect of stray coupling, allowing to use a selective group of cables to be used to connect emitter, receiver and conditioning and measuring unit.

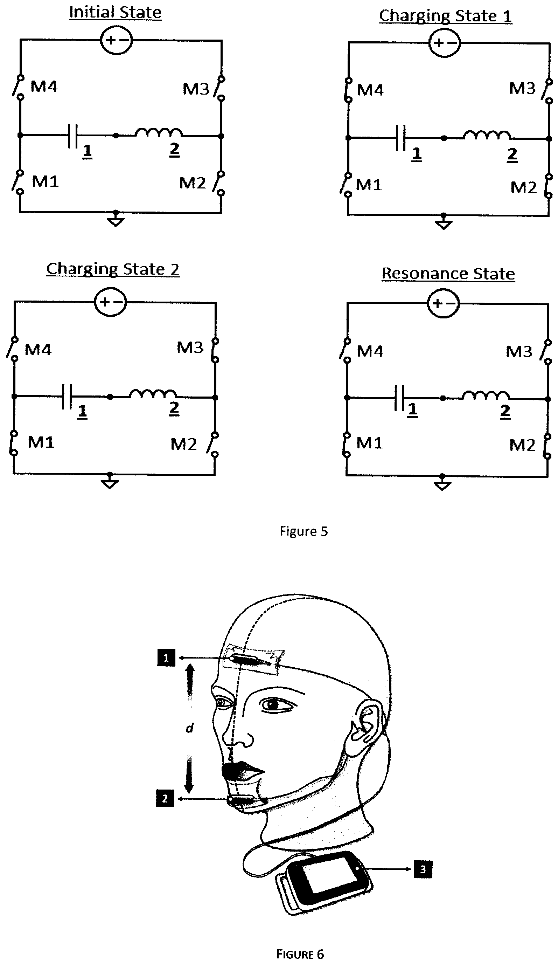

FIG. 4 describes these 4 states used by the invention and the corresponding position of the switches. FIG. 5 shows a schematic representation of each of the 4 possible states.

The emitter is arranged to produce a magnetic field by means of the resonant circuit formed by the inductance and the capacitor. The resonant circuit has an associated resonant frequency and the receiver is arranged to pick up at said resonant frequency the magnetic field emitted by the emitter and to provide the associated voltage level to the input of the detector. As illustrated in FIG 2a, The emitter produces a magnetic field from a series of pulses supplied at the energizing circuit. Thus this magnetic field is produced intermittently and each emission has a predetermined energy, in particular determined by the value of the inductance and the capacitor, the voltage level of the energizer and the number of charging pulses.

Fig 6. The device is then mounted on a support arranged to be applied onto the head of a living being so as to measure movements of the mouth. The operating parameters of the sensor are adjusted to produce a peak-to-peak measurement noise less than 0,1 mm for mouth openings up to 5 cm compared with the mouth closed position. The measurement reference can be placed anywhere along the median line of the face, above the upper lip, as shown in FIG. 6.

A common implementation of the present invention places this reference under the nose (A). Another common implementation places this reference on the forehead (B). Alternatively, the reference point can be placed inside the mouth, on the teeth, palate or gums. The measurement point of the sensor must be placed so as to monitor the movements of the mandible as well as possible. In the preferred implementation of the invention, it is placed in the hollow under the lower lip, where the relative movements between the bone structure and the skin are smallest. Alternatively, the measurement point can be situated at the point of the chin, under the chin, or inside the mouth, on the teeth or gums.

In the preferred embodiment of the invention, the two elements of the sensor are held in place by means of a comfortable harness, small in size and easily positioned. Supporting the reference point can be performed by any known means. Supporting the measuring point is more difficult. To minimize discomfort for the patient, structures situated close to the mouth and on the cheeks must be very light.

Moreover, effective support over the whole opening dynamics should be ensured without imposing any force on the mandible which would risk affecting the measurements.

Finally, independence as regards movements of rotation and inclination of the head forwards or backwards is essential. The preferred embodiment of the invention has a sensor structure of elongated shape provided with a link imposing a small traction from the hollow situated under the lower lip along a line passing under the ear lobe. Alternatively, an additional support for the sensor can be provided by a chin strap.

It should be understood that the present invention is not limited to the described embodiments and that variations can be applied without going outside of the scope of the appended claims.

## Claims

1. Distance measuring device comprising an emitter and a receiver,

i) wherein said emitter (1) comprises a resonance circuit , said resonance circuit having a resonance frequency ,said emitter being further arranged to produce a magnetic field emission intermittently, said emission having a maximum predefined energy value,

ii) said receiver being connected to a conditioning and measuring unit comprising a detector, said receiver being further arranged to pick up at said resonance frequency the magnetic field produced by the emitter, said receiver being further arranged to provide a first signal corresponding to the voltage level associated to said picked up magnetic field emission to the input of said detector,

iii) said detector being arranged to determine a residual energy value by correlation of said first signal with a second predetermined signal having a waveform representative of a signal to be picked up by the receiver, said second signal comprising a time window having a predetermined duration and comprising at least an initial sub-period and a final sub-period, said second signal being an alternating signal synchronized with the first signal and whereof the amplitude is attenuated during the initial and the final periods, said detector being arranged to determine based on the ratio between the maximum energy value and said residual energy value, a distance measurement signal representing the distance between said emitter and said receiver,

**characterized in that** said resonance circuit of the emitter comprises an inductance and a capacitance, said resonance circuit being further connected to said conditioning and measuring unit, comprising an energizing circuit, said energizing circuit comprising a first, a second, a third and a fourth transistor switch in series, said resonance circuit being connected to the energizing circuit, said capacitance of the resonance circuit being connected to the first and fourth switch of the energizing circuit on one side of the capacitance and to the inductance on the opposite side of the capacitance, said inductance of the resonance circuit being further connected to the second and third transistor switch, said energizing circuit being further connected to a voltage source between the fourth and third switches and connected to ground between the first and second transistor switches, said two circuits being arranged to switch between different charging states to create and amplify a magnetic field, generated by said resonance circuit, until the energy value of said magnetic field reaches the predefined maximum energy value, said emitter and said conditioning and measuring unit, said receiver and said conditioning and measuring unit being connected to each other by using a cable selected in the group consisting of shielded single core, coaxial, shielded twisted pair, twisted pair, ...

2. Distance measuring device according to claim 1, wherein said detector is arranged to implement said correlation with said second signal, which second signal is formed by said waveform representing the signal to be picked up as obtained in the absence of perturbation.

3. Distance measuring device according to claim 1, wherein said detector is arranged to implement said correlation with said second signal, which second signal is formed by said waveform representing a sinusoidal waveform in synchronization with the first signal itself multiplied by a time window function.

4. Distance measuring device as claimed in claim 1, wherein said detector is arranged to implement said correlation with said second signal, which second signal is formed by said waveform representing a square waveform in synchronization with the first signal.

5. Distance measuring device according to any of the claims 1 to 4, wherein said emitter is housed in a case and arranged to produce said magnetic field outside said case with a strength less than 2 mTesla, preferably less than 1mTesla, more preferably less than 1 $\mu$Tesla.

6. Distance measuring device according to any of the claims 1 to 5, wherein said emitter comprises an inductance and a capacitor connected in series with one another and connected by means of electrical conductors to an energizing circuit, said energizing circuit comprising a voltage source.

7. Distance measuring device as claimed in claim 6, wherein said energizing circuit is placed close to the inductance and the capacitor and forms an autonomous unit with respect to the receiver.

8. Sleep disorder detector comprising a distance measuring device as claimed in any of the claims 1 to 7, wherein said distance measuring device is encapsulated in a case provided for being placed directly on the head of a living being as to measure the movement of the mouth.

9. Sleep disorder detector as claimed in claim 8, wherein said detector comprises an analyzer having an input connected to the distance measuring device and arranged to receive said distance measurement, said analyzer being arranged to compare and match the measured signal with a collection of characteristic value sets describing movement patterns of the mouth of a living being, and to identify a characteristic set of movement pattern within said collection characteristic value sets describing movement patterns, producing a detection signal upon.

10. Sleep disorder detector as claimed in claim 8, wherein said detector is connected to an analyzer having an input connected to the distance measuring device and arranged to receive said distance measurement, said analyzer being arranged to compare and match the measured signal with a collection of characteristic value sets describing movement patterns of the mouth of a living being, and to identify a characteristic set of movement pattern within said collection characteristic value sets describing movement patterns, producing a detection signal upon.

11. Sleep disorder detector as claimed in claim 9 and claim 10, wherein said characteristic value sets comprises a first characteristic set of characteristic values describing a sudden closing of the mouth, a second characteristic set of characteristic values indicating a slow opening of the mouth, a third characteristic set of characteristic values indicating a slow closing of the mouth, and a fourth characteristic set of characteristic values indicating an increase in the amplitude of the signal at the breathing frequency followed by a decrease in the signal at the breathing frequency.

12. Sleep disorder detector as claimed in any of the claims 9 to 11, wherein said detector will add the distance measure signal to the characteristic set of characteristic values, to be used and to improve the matching to this pattern in the future.

13. Sleep disorder detector as claimed in any of the claims 9 to 12, wherein said analyzer is further arranged to produce a specific sleep disorder signal upon recognition of a specific sequence of any of the first, second, third and a fourth characteristic set of characteristic values

14. Sleep disorder detector as claimed in claim 13, this detector comprising a decision element using said detection signal to provide an indication of insufficient, correct or excessive treatment for the targeted sleep disorders.

**15.** Movement analyzer comprising a distance measuring device as claimed in any of the claims 1 to 7, wherein said device is mounted on a support arranged to be applied around a joint of a living being as so to measure the characteristics and/or statistics of the movements of this joint.

**16.** Method for measuring a distance for identifying a sleep disorder comprising the steps of :

i) Production of a magnetic field intermittently having a maximum predefined energy value by an emitter having a resonance circuit having a resonance frequency,
ii) picking up by said receiver at said resonance frequency the magnetic field produced by the emitter, and providing the associated voltage level to the input of said conditioning and measuring unit comprising said detector, to whom said receiver is connected,
iii) Determining by said detector based on the ratio between the maximum energy value and the residual energy value, a distance measurement signal representing the distance between said emitter and said receiver, said determining step of said distance measurement signal being done by correlation of said first signal with a second predetermined signal having a waveform representative of a signal to be picked up by the receiver, said second signal comprising a time window having a predetermined duration and comprising at least an initial sub-period and a final sub-period, said second signal being an alternating signal synchronized with the first signal and whereof the amplitude is attenuated during the initial and the final periods,

**characterized in that** said method further comprises a step of alternating opposite charging states of the resonance circuit, thereby creating and amplify a magnetic field, generated by said resonance circuit, until the energy value of said magnetic field reaches the predefined maximum energy value, said emitter and said conditioning and measuring unit, said receiver and said conditioning and measuring unit being connected to each other by using a cable selected in the group consisting of shielded single core, coaxial, shielded twisted pair, twisted pair, ...

**17.** Method for measuring a distance for identifying a sleep disorder according to claim 16, further comprising a multiplication and integration with said second signal formed by said waveform representing the signal to be picked up as obtained in the absence of perturbation by said detector.

**FIGURE 1**

**FIGURE 2**

3V3

PWM4 —— M4    MOS "P"    M3 —— PWM3

PWM1 —— M1    MOS "N"    M2 —— PWM2

GND

FIGURE 3

| | SWITCH (M1) | SWITCH (M2) | SWITCH (M3) | SWITCH (M4) |
|---|---|---|---|---|
| INITIAL STATE | OFF | OFF | OFF | OFF |
| CHARGING 1 | OFF | ON | OFF | ON |
| CHARGING 2 | ON | OFF | ON | OFF |
| RESONANCE | ON | ON | OFF | OFF |

FIGURE 4

## Initial State

## Charging State 1

## Charging State 2

## Resonance State

Figure 5

FIGURE 6

Europäisches
Patentamt

European
Patent Office

Office européen
des brevets

## EUROPEAN SEARCH REPORT

Application Number

EP 18 24 8244

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | US 2007/273366 A1 (ANSAY PIERRE [BE] ET AL) 29 November 2007 (2007-11-29) * the whole document * ----- | 1-17 | INV. A61B5/08 A61B5/107 A61B5/113 |
| Y | EP 2 808 975 A1 (FRIWO GERÄTEBAU GMBH [DE]) 3 December 2014 (2014-12-03) * paragraph [0041] - paragraph [0042] * ----- | 1-17 | A61B5/00 |

TECHNICAL FIELDS
SEARCHED      (IPC)

A61B

The present search report has been drawn up for all claims

3

| Place of search | Date of completion of the search | Examiner |
|---|---|---|
| The Hague | 27 June 2019 | Van Dop, Erik |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 18 24 8244

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2019

| Patent document cited in search report | | Publication date | Patent family member(s) | | Publication date |
|---|---|---|---|---|---|
| US 2007273366 | A1 | 29-11-2007 | AT | 440262 T | 15-09-2009 |
| | | | AU | 2005206655 A1 | 04-08-2005 |
| | | | CA | 2553590 A1 | 04-08-2005 |
| | | | CN | 1973177 A | 30-05-2007 |
| | | | EP | 1555505 A1 | 20-07-2005 |
| | | | EP | 1716387 A1 | 02-11-2006 |
| | | | US | 2007273366 A1 | 29-11-2007 |
| | | | WO | 2005071353 A1 | 04-08-2005 |
| EP 2808975 | A1 | 03-12-2014 | CN | 104218684 A | 17-12-2014 |
| | | | DK | 2808975 T3 | 14-01-2019 |
| | | | EP | 2808975 A1 | 03-12-2014 |
| | | | ES | 2700533 T3 | 18-02-2019 |
| | | | HK | 1203701 A1 | 30-10-2015 |
| | | | HU | E041440 T2 | 28-05-2019 |
| | | | JP | 6035282 B2 | 30-11-2016 |
| | | | JP | 2014233197 A | 11-12-2014 |
| | | | PL | 2808975 T3 | 29-03-2019 |
| | | | US | 2014354065 A1 | 04-12-2014 |

EPO FORM P0459

For more details about this annex : see Official Journal of the European Patent Office, No. 12/82

**EP 3 673 802 A1**

## REFERENCES CITED IN THE DESCRIPTION

**Patent documents cited in the description**

- US 20070273366A1 A
- DE 4114398
- US 20070273366 A1
- US 20070273366 A